# EUROPEAN PATENT SPECIFICATION

(11) **EP 4 346 968 B1**
(45) Date of publication and mention of the grant of the patent: **17.09.2025**
(21) Application number: 22726152.6
(22) Date of filing: 13.04.2022
(51) Int. Cl.: A61M 16/10, A61M 16/04

(54) **HEAT AND MOISTURE EXCHANGE DEVICE**
WÄRME- UND FEUCHTIGKEITSTAUSCHER
DISPOSITIF D'ÉCHANGE DE CHALEUR ET D'HUMIDITÉ

(30) Priority: 24.05.2021 GB 202107383
(43) Date of publication of application: 10.04.2024
(73) Proprietor: ICU Medical International Limited, Lower Pemberton Ashford, Kent TN25 4BF (GB)
(72) Inventor: TUPPER, Steven, Mark, Hythe, Kent CT21 5XW (GB); BATEMAN, Timothy, Hythe, Kent TN29 0QD (GB); JEFFREY, Andrew, Thomas, Romney Marsh, Kent TN29 0RB (GB); WOOSNAM, Christopher, John, London SW12 8NY (GB)
(74) Representative: Flint, Jonathan McNeill
(86) International application number: PCT/GB2022/000048
(87) International publication number: WO 2022/248820

(56) References cited:
- EP-B1- 0 651 665
- EP-B1- 0 696 925
- DE-U1- 202005 006 605
- US-A1- 2015 343 164
- US-A1- 2019 351 173
- US-B1- 6 792 946

## Description

This invention relates to HME devices of the kind including an outer housing and at least one HME element mounted in the housing, the HME device being arranged to fit with a cooperating male tapered connector on a tracheostomy tube.

Where a patient breathes through a tube inserted in the trachea, such as a tracheostomy or endotracheal tube, gas flow to the bronchi is not warmed and moistened by passage through the nose. Unless the gas is warmed and moistened in some way it can cause damage and discomfort in the patient's throat. The gas can be conditioned by a humidifier in the ventilation circuit but, most conveniently, a heat and moisture exchange device (HME) is used. HMEs are small, lightweight devices including one or more HME exchange elements. When the patient exhales, gas passes through the exchange element and gives up a major part of its heat and moisture to the element. When the patient inhales, gas passes through the exchange element in the opposite direction and takes up a major part of the heat and moisture in the exchange element so that the gas inhaled by the patient is warmed and moistened. These HMEs are low cost and are disposable after a single use. They can be connected in a breathing circuit or simply connected to the machine end of a tracheal tube and left open to atmosphere where the patient is breathing spontaneously. HMEs can be used with other breathing devices such as face masks.

HMEs are sold by Smiths Medical International Limited of Ashford, Kent, England under the Thermovent name (Thermovent is a registered trade mark of Smiths Medical International Limited), by Hudson RCI AB under the TrachVent name (TrachVent is a registered trade mark of Hudson RCI AB), by DAR, Medisize, Intersurgical and other manufacturers. Examples of HMEs are described in GB2391816, WO01/72365, US5505768, SE516666, US3881482, DE20302580, DE20114355U, WO97/01366, US2002/0157667, US6422235, EP1208866, US4971054, EP1699515, US5035236, EP535016, US5647344, GB2267840, EP856327, EP1699515, US7363925, WO15/107320, US2008/0099013 and GB2540456.

Further examples of HME's are described in US 2019/351173 A1, DE 20 2005 006605 U1, EP 0 651 665 B1, US 2015/343164 A1, EP 0 696 925 B1 and US 6 792 946 B1. Conventional HMEs have a female connection port that fits onto the outside of the usual male 15mm connector at the machine end of a tracheostomy tube or the like. The HME may have an axial configuration with a cylindrical outer housing containing the HME element and having the female connection port at one end and a male connection port at the opposite end, which may be connected to a breathing circuit or may be left open if the patient is breathing unaided. The housing of the HME, therefore, projects outwardly and generally axially beyond the machine end of the tracheostomy tube.

The "Thermovent T" HME sold by Smiths Medical has a different, T-shape configuration with two HME elements mounted at opposite ends of a straight tubular housing extending transversely of the connection port by which the device is fitted onto a tracheostomy tube or the like. The tubular housing for the HME elements may be curved to follow the anatomical profile of the neck, as described in EP1888157.

Both the axial and T-shape configuration HMEs have the HME element or elements contained in a part of the HME housing that projects outwardly of the tracheostomy tube or other breathing device. Where the patient is breathing spontaneously and is not connected with a breathing circuit the HME increases the distance of the protrusion from the stoma so is more conspicuous and more likely to catch on clothing or bedding causing displacement of the tracheostomy tube and discomfort to the patient. Conventional HMEs also have a disadvantage of introducing additional deadspace to the gas passage to and from the patient. The tapered slip fit between the HME coupling and the machine end connector on the breathing device means that the HME must be twisted relative to the connector, which inevitably leads to some twisting motion of the tracheal tube itself when the HME is removed and replaced. This can cause discomfort to the patient.

The invention is defined in the appended claims. It is an object of the present disclosure to provide an alternative HME device, an HME element, and an assembly of a breathing device and an HME device.

According to one aspect of the present disclosure there is provided an HME device of the above-specified kind, characterised in that the HME device is configured such that a major part of the length of the HME element is arranged to extend inside the cooperating connector on the tube, that the outer housing of the HME device has a tapered inner surface suitable to fit on the male tapered outside of the cooperating connector, and that the outside of the HME element is spaced from the inner surface of the outer housing at least along its patient end such as to define an annular recess at the patient end of the device in which at least the machine end of the cooperating connector can be received such that at least the patient end of the HME element is located within the cooperating connector.

The HME element may be removable from the outer housing of the HME device from the machine end of the outer housing when the HME device is fitted on a connector. The HME element may be retained with the outer housing by a clip fitting, the clip fitting being releasable from the outer housing by pulling away from the outer housing without the need to twist the element relative to the housing. The HME device may be joined to the machine end of an inner cannula so that the HME device and inner cannula can be removed and replaced as a single unit.

According to another aspect of the present disclosure there is provided an HME element for an HME device according to the above one aspect of the present disclosure.

According to a further aspect of the present disclosure there is provided an assembly of a tracheostomy tube and an HME device according to the above one aspect of the present disclosure, characterised in that the outer housing of the HME device is fitted on the outside of the connector with a part at least of the HME element extending within the connector.

An HME device, an HME element and an assembly of an HME device on a tracheostomy tube all according to the present disclosure will now be described, by way of example, with reference to the accompanying drawings, in which:
- Figure 1: is a perspective view of an assembly of a tracheostomy tube with an HME device fitted on its machine end;
- Figure 2: is a cross-sectional side elevation view to an enlarged scale of the machine end of the assembly in Figure 1; and
- Figure 3: is a perspective view of the assembly with the HME element removed from the HME device outer housing.

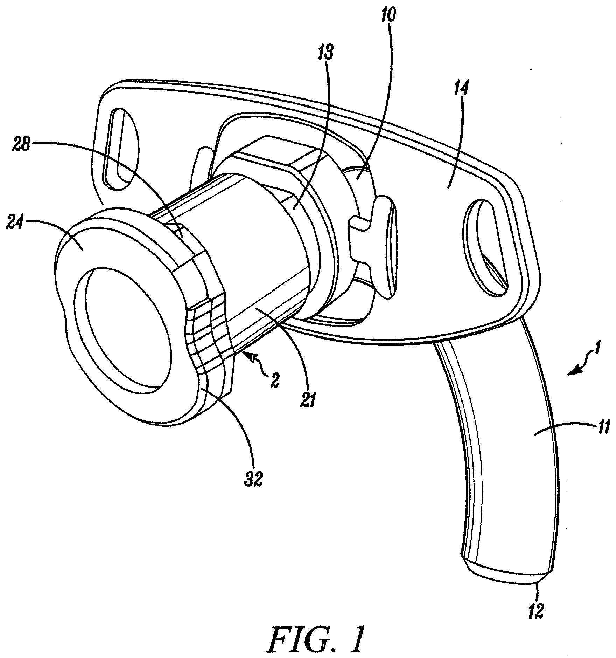

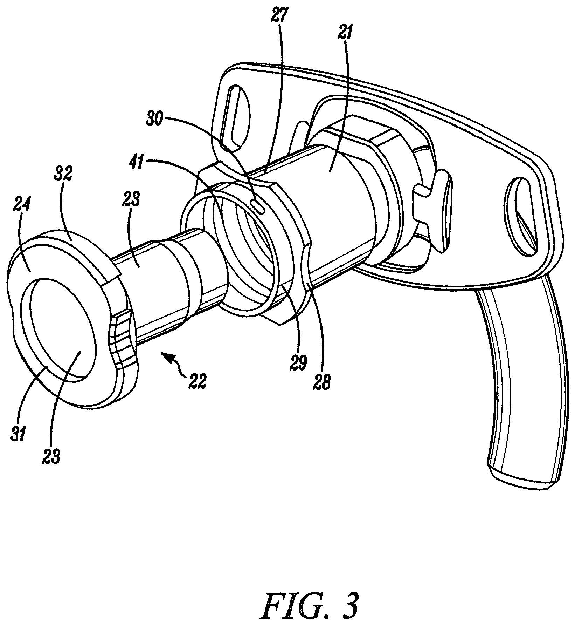

With reference first to Figures 1 and 3 there is shown an assembly of a tracheostomy tube 1 with an HME device 2 fitted on its rear, machine end 10. The tracheostomy tube 1 can be conventional, having a curved shaft 11 that is open at its forward, patient end 12. The rear, machine end 10 of the shaft 11 is joined with a conventional 15mm tapered male connector 13 and a radially extending neck flange 14.

The HME device 2 differs from previous HMEs in that it is arranged so that a major part of the length of its HME element 20 is contained within the length of the tracheostomy tube connector 13. In particular, the HME 2 comprises an outer housing 21 and a removable HME fitting 22 formed of an HME element 23 and an end cap 24. The outer housing 21 has a tubular or cylindrical shape with a circular section and is moulded of a rigid plastics. Internally, the housing 21 has a tapered inner surface 26 that tapers gradually to a larger diameter at its patient end and is configured to make a tapered slip fit on the outside of the male connector 13 on the tracheostomy tube 1. Towards its rear, machine end 27 the housing 21 has a radially projecting knurled flange 28 spaced from the machine end of the housing by a short axially-projecting sleeve 29. The outer surface of the sleeve 29 is moulded with several radially-projecting pips or projections 30 spaced around the sleeve and that act as part of a clip fitting. The end cap 24 has a circular opening 31 occupying most of the diameter of the cap. The cap 24 has a forwardly-projecting collar 32 with an internal diameter equal to the external diameter of the sleeve 29. The inner surface of the collar 32 also has an annular groove 33 around its circumference the width and depth of which is sufficient to receive the pips 30 on the sleeve 29. Engagement of the pips 30 in the groove 33 is sufficient to retain the cap 24 on the housing 21.

The end cap 24 is securely bonded to the machine end of the HME element 23. The HME element 23 has a circular cross section and is generally cylindrical. The HME element 23 has an enlarged machine end portion 34 with an outwardly flared machine end section 35, and a central section 36 of constant circular section extending for the major part of the length of the element 23. A short forward, patient end portion 37 has a reduced diameter and connects with the central section 36 by a tapered region 38. The element 23 is longer than the outer housing 21 so that the patient end portion 37 projects beyond the patient end of the outer housing. The enlarged machine end portion 34 is a close sealing fit within the inside of the machine end of the outer housing 21. That part of the HME element 23 forwardly of the machine end portion 34 is spaced along its length from the inside of the outer housing 21 by an annular recess 40. The dimensions and shape of the annular recess 40 are selected to receive the major part of the length of the connector 13 on the tracheostomy tube 1. In this way, the outer surface of the HME element 23 is a close sealing fit within the forward, patient end part of the connector 13. Towards its rear, machine end the connector 13 is enlarged internally slightly and is formed with an internal sealing ring 41 adapted sealingly to contact the outside of the element 23.

The HME element 23 could be of any conventional kind such as a coiled strip of corrugated paper treated with a hygroscopic salt or a foamed plastics coated with a hygroscopic substance. The HME element 23 could include a filter, such as in the enlarged machine end portion 34. The HME element 23 could be coated around its curved surface with a flexible plastics material to enhance the seal between the outside of the element and the inside of the connector 13.

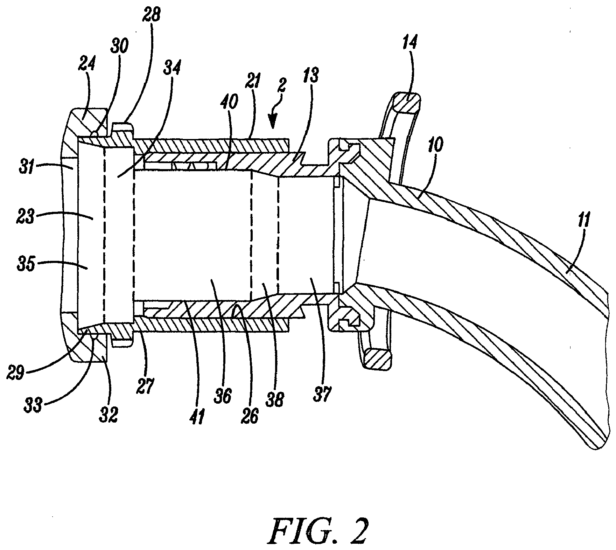

With the HME device 2 fitted on the tracheostomy tube 1 in the manner shown in Figures 1 and 2 the HME element 23 occupies substantially the entire length of the interior of the connector 13. When the patient inhales air is drawn in through the HME element 23, through the HME device 2 and connector 13 and into the bore of the tracheostomy tube 1. Exhaled gas flows back in the opposite direction, giving up most of its heat and moisture to the HME element 23 so that this is transferred to the next inspiratory breath.

By arranging the HME element 23 to extend inside the connector 13 on the tracheostomy tube 1 the length of the HME projecting from the tube can be substantially reduced, making the overall assembly less bulky than conventional HMEs. This makes the tracheostomy assembly itself less conspicuous. The smaller profile also reduces the risk of catching the machine end of the assembly on bedding or clothing and the like. By moving the centre of mass of the HME closer to the patient's neck the leverage on the tube contributed by the HME can be reduced compared with conventional HMEs. Another advantage of extending the HME element inside the connector is that the deadspace in the assembly can be reduced. In conventional assemblies the deadspace includes the internal volume of the connector on the tube and the volume within the connected HME housing up to the HME element itself. By contrast, in the arrangement of the present disclosure the deadspace is only that contributed by the internal volume of the tube on the patient side of the HME element. By reducing the deadspace the volume of exhaled air returned to the lungs on inspiration that has not passed through the HME element is reduced.

The HME device 2 can be fitted and removed, in its entirety, on the connector 13 by a twist slip action. However, instead of removing the device 2 entirely, when a replacement is needed, it is only necessary to remove the HME element 23 and its associated end cap 24. This is done simply by pulling the end cap 24 away from the outer housing 21 and the connector 13 without any twist action, as shown in Figure 3. This releases the end cap 24 from the pips 30 on the outer housing 21 of the HME device 2. This arrangement has the advantage of avoiding the need to apply any twist torque to the tube 1, thereby reducing trauma and discomfort to the patient. In this way the HME element can be removed and replaced every 24 hours, or as necessary with less discomfort to the patient. If access is needed to the tube, such as for suctioning, this can also be done by removing just the central HME element and leaving the outer HME housing in place. Another advantage is that the replaceable element reduces the amount of plastics used since the outer housing can be reused several times. This reduces overall costs and also reduces the amount of waste plastics produced, thereby reducing environmental damage, and saving some of the expense of disposing of clinical waste. A tracheostomy tube or the like arranged to receive an HME of the kind of the present disclosure can still be connected to a conventional female tapered connector at the end of a gas supply tube when ventilation is required, simply by removing the entire HME.

The HME could be joined to the machine end of an inner cannula adapted to extend along the bore of the tracheostomy tube, the inner cannula being of the kind that is removed and replaced to avoid the build-up of secretions in the outer tube. In such an arrangement the HME would be removed and replaced as a single unit with the inner cannula.

It is not essential for the HME element to be removable from the outer housing since it could be permanently attached with the outer housing so that the entire device must be removed when it needs to be replaced.

The present disclosure is not confined to HME devices for use on tracheostomy tubes but could be used on other breathing devices such as endotracheal tubes, laryngeal masks, and face masks.

## Claims

1. An HME device (2) including an outer housing (21) and at least one HME element (23) mounted in the housing, the HME device being arranged to fit with a cooperating male tapered connector (13) on a tracheostomy tube (1), wherein the HME device (2) is configured such that a major part of the length of the HME element (23) is arranged to extend inside the cooperating connector (13) on the tube (1), **characterised in that** the outer housing (21) of the HME device (2) has a tapered inner surface (26) suitable to fit on the male tapered outside of the cooperating connector (13), and that the outside of the HME element (23) is spaced from the inner surface of the outer housing (21) at least along its patient end such as to define an annular recess (40) at the patient end of the device (2) in which at least the machine end of the cooperating connector (13) can be received such that at least the patient end of the HME element (23) is located within the cooperating connector (13).

2. An HME device according to Claim 1, **characterised in that** the HME element (23) is removable from the outer housing (21) of the HME device (2) from the machine end of the outer housing (21) when the HME device is fitted on a connector (13).

3. An HME device according to Claim 2, **characterised in that** the HME element (23) is retained with the outer housing (21) by a clip fitting (29, 30), and that the clip fitting is releasable from the outer housing by pulling away from the outer housing (21) without the need to twist the element (23) relative to the housing (21).

4. An HME device according to any one of the preceding claims, **characterised in that** the HME device (2) is joined to the machine end of an inner cannula so that the HME device and inner cannula can be removed and replaced as a single unit.

5. An assembly of a tracheostomy tube (1) and an HME device (2) according to any one of Claims 1 to 4, **characterised in that** the outer housing (21) of the HME device (2) is fitted on the outside of the connector (13) with a part at least of the HME element (23) extending within the connector (13).

## Patentansprüche

1. HME-Vorrichtung (2) umfassend ein äußeres Gehäuse (21) und mindestens ein in dem Gehäuse angebrachtes HME-Element (23), wobei die HME-Vorrichtung so ausgebildet ist, dass sie mit einem männlichen, sich verjüngenden zusammenwirkenden Verbindungsstecker (13) eines Tracheostomietubus (1) zusammenpasst, wobei die HME-Vorrichtung (2) so konfiguriert ist, dass ein Großteil der Länge des HME-Elements (23) so ausgebildet ist, dass es sich in das Innere des zusammenwirkenden Verbindungssteckers (13) an der Trachealkanüle (1) erstreckt, **dadurch gekennzeichnet, dass** das äußere Gehäuse (21) der HME-Vorrichtung (2) eine sich verjüngende Innenfläche (26) aufweist, die angepasst ist, auf die sich verjüngende Außenseite des zusammenwirkenden Verbindungssteckers (13) zu passen, und dass die Außenseite des HME-Elements (23) von der Innenfläche des äußeres Gehäuses (21) zumindest längs seines Patientenendes beabstandet ist, um eine ringförmige Aussparung (40) am patientenseitigen Ende der Vorrichtung (2) zu definieren, in der zumindest das maschinenseitige Ende des zusammenwirkenden Verbindungssteckers (13) aufgenommen werden kann, so dass zumindest das patientenseitige Ende des HME-Elements (23) innerhalb des zusammenwirkenden Verbindungssteckers (13) angeordnet ist.

2. HME-Vorrichtung nach Anspruch 1, **dadurch gekennzeichnet, dass** das HME-Element (23) von dem äußeren Gehäuse (21) der HME-Vorrichtung (2) vom maschinenseitigen Ende des äußeren Gehäuses (31) entfernbar ist, wenn die HME-Vorrichtung auf einen Verbindungsstecker (13) aufgesetzt ist.

3. HME-Vorrichtung nach Anspruch 2, **dadurch gekennzeichnet, dass** das HME-Element (23) durch eine Klemmbefestigung (29, 30) von dem äußeren Gehäuse (21) gehalten wird, und dass die Klemmbefestigung durch Abziehen vom äußeren Gehäuse (21) von dem äußeren Gehäuse lösbar ist, ohne dass das Element (23) relativ zum Gehäuse verdreht werden muss.

4. HME-Vorrichtung nach einem der vorhergehenden Ansprüche, **dadurch gekennzeichnet, dass** die HME-Vorrichtung (2) mit dem maschinenseitigen Ende einer Innenkanüle verbunden ist, so dass die HME-Vorrichtung und die Innenkanüle als eine Einheit entfernt und ersetzt werden können.

5. Baugruppe aus einer Trachealkanüle (1) und einer HME-Vorrichtung (2) nach einem der Ansprüche 1 bis 4, **dadurch gekennzeichnet, dass** das äußeres Gehäuse (21) der HME-Vorrichtung (2) an der Außenseite des Verbindungssteckers (13) angebracht ist, wobei sich zumindest ein Teil des HME-Elements (23) innerhalb des Konnektors (13) erstreckt.

## Revendications

1. Dispositif HME (2) comprenant un boîtier extérieur (21) et au moins un élément HME (23) monté dans le boîtier, le dispositif HME étant conçu pour s'adapter à un connecteur conique mâle coopérant (13) sur un tube de trachéotomie (1), dans lequel le dispositif HME (2) est configuré de telle sorte qu'une grande partie de la longueur de l'élément HME (23) est conçue pour s'étendre à l'intérieur du connecteur coopérant (13) sur le tube (1), **caractérisé en ce que**, le boîtier extérieur (21) du dispositif HME (2) a une surface intérieure conique (26) adaptée pour s'adapter à l'extérieur conique mâle du connecteur coopérant (13), et que l'extérieur de l'élément HME (23) est espacé de la surface intérieure du boîtier extérieur (21) au moins le long de son extrémité patient de manière à définir un évidement annulaire (40) à l'extrémité patient du dispositif (2) dans lequel au moins l'extrémité machine du connecteur coopérant (13) peut être reçue de telle sorte qu'au moins l'extrémité patient de l'élément HME (23) soit située à l'intérieur du connecteur coopérant (13).

2. Dispositif HME selon la revendication 1, **caractérisé en ce que** l'élément HME (23) est amovible du boîtier extérieur (21) du dispositif HME (2) à partir de l'extrémité machine du boîtier extérieur (21) lorsque le dispositif HME est monté sur un connecteur (13).

3. Dispositif HME selon la revendication 2, **caractérisé en ce que** l'élément HME (23) est retenu avec le boîtier extérieur (21) par un raccord à clip (29, 30), et **en ce que** le raccord à clip peut être libéré du boîtier extérieur en le tirant hors du boîtier extérieur (21) sans qu'il soit nécessaire de tordre l'élément (23) par rapport au boîtier (21).

4. Dispositif HME selon l'une quelconque des revendications précédentes, **caractérisé en ce que** le dispositif HME (2) est relié à l'extrémité machine d'une canule interne de sorte que le dispositif HME et la canule interne peuvent être retirés et remplacés comme une seule unité.

5. Ensemble d'un tube de trachéotomie (1) et d'un dispositif HME (2) selon l'une quelconque des revendications 1 à 4, **caractérisé en ce que** le boîtier extérieur (21) du dispositif HME (2) est monté à l'extérieur du connecteur (13) avec une partie au moins de l'élément HME (23) s'étendant à l'intérieur du connecteur (13).
